(19) Europäisches Patentamt
European Patent Office
Office européen des brevets

(11) Veröffentlichungsnummer: 0 206 147
B1

(12) # EUROPÄISCHE PATENTSCHRIFT

(45) Veröffentlichungstag der Patentschrift: 28.02.90

(51) Int. Cl. 5: C 07 C 205/06, C 07 C 205/12, C 07 C 201/12

(21) Anmeldenummer: 86108035.6

(22) Anmeldetag: 12.06.86

(54) **Verfahren zur Herstellung von 4,4'-Dinitrodibenzylen.**

(30) Priorität: 28.06.85 DE 3523204

(43) Veröffentlichungstag der Anmeldung: 30.12.86 Patentblatt 86/52

(45) Bekanntmachung des Hinweises auf die Patenterteilung: 28.02.90 Patentblatt 90/09

(84) Bennante Vertragsstaaten: CH DE FR GB IT LI

(56) Entgegenhaltungen:
DE-A-2 051 167
US-A-2 965 681
US-A-4 247 724

(73) Patentinhaber: BAYER AG
D-5090 Leverkusen 1 Bayerwerk (DE)

(72) Erfinder: Kopp, Richard, Dr.
Bilharzstrasse 15
D-5000 Koeln 80 (DE)
Erfinder: Grögler, Gerhard, Dr.
von Diergardtstrasse 48
D-5090 Leverkusen (DE)
Erfinder: König, Klaus, Dr.
Heymannstrasse 50
D-5090 Leverkusen (DE)
Erfinder: Schmidt, Manfred, Dr.
Zeisigstrasse 5
D-4047 Dormagen (DE)

EP 0 206 147 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft ein Verfahren zur Herstellung von 4,4'-Dinitrodibenzylen aus 4-Nitrotoluolen durch oxidative Dimerisierung.

Die Herstellung von 4,4'-Dinitrodibenzyl aus 4-Nitrotoluol durch oxidative Dimerisierung mit Luftsauerstoff in stark alkalischer Lösung ist bekannt und wird beispielsweise in Chem. Berichte 26, 2232 (1893), J. Chem. Soc. 91, 2079 (1907), J. Amer. Chem. Soc. 75, 1325 (1953), Org. Synth. Coll. Vol. IV, 367 (1963), beschrieben. Auch der Mechanismus der genannten Reaktion ist eingehend untersucht worden (siehe z. B. J. Amer. Chem. Soc. 77, 3265 (1955), Abstr. of Papers, 135th Meeting Amer. Chem. Soc., April 5 - 14, 1960, S. 6 - 25; Monatsh. Chem. 34, 1011 (1913), J. Amer. Chem. Soc. 84, 4153 (1962) und J. Amer. Chem. Soc. 88, 5491 (1966)).

Ein verbessertes Verfahren zur Herstellung von 4,4'-Dinitrodibenzyl aus 4-Nitrotoluol durch oxidative Dimerisierung mit Sauerstoff in alkalischer Lösung wird beschrieben in J. Org. Chem. 26, 4162 (1961) und in der US-PS-2 965 681. Bei diesen Verfahren wird in Gegenwart bestimmter Amine oder Ether gearbeitet.

Weiterhin ist aus der DE-OS-2 051 167 ein Verfahren zur Herstellung von 4,4'-Dinitrodibenzyl bekannt, bei dem die oxidative Dimerisierung mit Luft oder Sauerstoff in methanolischer oder ethanolischer NaOH- oder KOH-Lösung durchgeführt wird und bei dem in einem speziellen Reaktor gearbeitet wird.

Die oxidative Dimerisierung in stark alkalischer Lösung mit Luft oder Sauerstoff ist das bisher einzige bekannte Verfahren zur Herstellung von technischen Mengen an Dinitrodibenzyl. Andere Methoden, wie die Umsetzung von 4-Nitrotoluol mit 0,5 mol Oxalsäurediethylester und 1 mol Natriummethylat (Chem. Ber. 30, 1053 (1897), Ann. Chem. 436, 56 (1924)), die Umsetzung von 4-Nitrobenzylchlorid mit alkalischer Stannat-Lösung (DRP 39 381), die Einwirkung von Quecksilberoxid auf in Chloroform gelöstes α,α-Bis[4-nitrobenzyl]hydrazin (Chem. Ber. 33, 2710 (1910)) oder die Nitrierung von Dibenzyl (J. Amer. Chem. Soc. 52, 5040 (1930), J. Chem. Soc. Jap. Ind. Chem. Sect. 61, 469 (1958)) sind ebenfalls bekannt, fanden aber bisher kein technisches Interesse.

Die obengenannten Verfahren zur Herstellung von 4,4'-Dinitrodibenzyl durch Oxidation von 4-Nitrotoluol mit Luft oder Sauerstoff sind jedoch mit wesentlichen Nachteilen behaftet. Zur Herstellung der reaktiven Zwischenstufe aus dem 4-Nitrotoluol werden Basenmengen von mindestens 4 bis 5 mol, bevorzugt sogar 10 mol Base pro mol 4-Nitrotoluol eingesetzt. Weiterhin wird die Umsetzung üblicherweise bei sehr geringen Nitrotoluolkonzentrationen durchgeführt (ca. 5 %-ige Lösungen), da sonst die Reaktionszeiten zu lang werden. Doch selbst unter diesen Bedingungen sind für die Luftoxidation Reaktionszeiten von etwa 6 bis 8 Stunden erforderlich. Dies bedeutet jedoch schlechte Raum/Zeitausbeuten, was zu Lasten der Wirtschaftlichkeit der erwähnten Verfahren geht. Weiterhin ist es erforderlich, um die Bildung von 4,4'-Dinitrostilben zu vermeiden, die Umsetzung bei niedrigeren Temperaturen, d.h. mit Kühlung, durchzuführen. Nachteilig bei den genannten Verfahren ist außerdem, daß das Produkt in sehr fein verteilter Form anfällt und daher die Filtration des Produkte mit großen technischen Problemen behaftet ist. Zusätzlich fallen große Mengen intensiv gefärbter Filtrate an, deren Aufbereitung ebenfalls einen erheblichen technischen Aufwand erfordert.

Es wurde nun ein Verfahren zur Herstellung von 4,4'-Dinitrodibenzylen der allgemeinen Formel

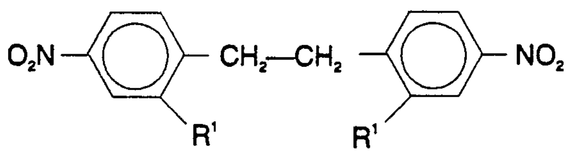

(I),

worin

$R^1$ für Wasserstoff, Alkyl, Aryl, Alkaryl, Aralkyl oder Halogen steht,

gefunden, das dadurch gekennzeichnet ist, daß man 4-Nitrotoluole der allgemeinen Formel

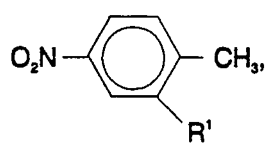

(II),

worin

$R^1$ die obengenannte Bedeutung besitzt,

in Gegenwart eines organischen Lösungs- und/oder Verdünnungsmittels mit einem Alkali- und/oder Erdalkalialkoholat umsetzt und anschließend das Reaktionsgemisch mit einer wäßrigen Lösung von unterhalogenigen Säuren und/oder

deren Salzen oder mit Chlor, Brom oder einer wäßrigen Lösung von Wasserstoffperoxid und/oder dessen Salzen oder mit anorganischen oder organischen Persäuren und/oder deren Salzen behandelt.

Als Alkylreste in der Formel (I) seien solche mit 1 bis 18 Kohlenstoffatomen, bevorzugt 1 bis 5 Kohlenstoffatomen, genannt, wie Methyl, Ethyl, n-Propyl, iso-Propyl, n-Butyl, sek.-Butyl, iso-Butyl, tert.-Butyl, Pentyl, Hexyl 2-Ethylhexyl, Decyl, Dodecyl, Octadecyl und Cyclohexyl, bevorzugt Methyl und Ethyl; als Arylreste solche mit 6 bis 20 Kohlenstoffatomen, bevorzugt 6 bis 10 Kohlenstoffatomen, wie Phenyl, Chlorphenyl, Bromphenyl und Naphthyl, bevorzugt Phenyl und Naphthyl; als Alkarylreste solche mit 7 bis 20 Kohlenstoffatomen, bevorzugt 7 bis 10 Kohlenstoffatomen, wie p-Tolyl, m-Tolyl, o-Tolyl, Ethylphenyl, Propylphenyl und Isopropylphenyl, bevorzugt p-Tolyl; als Aralkylreste solche mit 7 bis 20 Kohlenstoffatomen, bevorzugt 7 bis 10 Kohlenstoffatomen, wie Benzyl, Phenethyl, 3-Phenylpropyl und 2-Phenylpropyl, bevorzugt Benzyl und als Halogene F, Cl, Br, bevorzugt Cl.

Als Ausgangsverbindungen der allgemeinen Formel (II) kommen z. B. in Betracht: 4-Nitrotoluol, 1,2-Dimethyl-4-nitrobenzol, 2-Ethyl-4-nitrotoluol, 2-Propyl-4-nitrotoluol, 2-Phenyl-4-nitrotoluol, 2-Benzyl-4-nitrotoluol, 2-Fluor-4-nitrotoluol, 2-Chlor-4-nitrotoluol und 2-Brom-4-nitrotoluol, bevorzugt 4-Nitrotoluol, 1,2-Dimethyl-4-nitrobenzol, 2-Ethyl-4-nitrotoluol und 2-Chlor-4-nitrotoluol.

Als organische Lösungs- und/oder Verdünnungsmittel sind alle Lösungsmittel- und/oder Verdünnungsmittel geeignet, die eine zur Umsetzung mit 4-Nitrotoluol genügende Löslichkeit für das 4-Nitrotoluol und die Alkoholat-Base aufweisen. Werden wäßrige Lösungen von Oxidationsmitteln für die oxidative Dimerisierung eingesetzt, dann sollten die organischen Lösungsmittel eine Wasserlöslichkeit von $\geq$ 2 %, vorzugsweise $\geq$ 5 %, besonders bevorzugt $\geq$ 10 % aufweisen. Sie sollten darüber hinaus keine allzugroße Löslichkeit für das entstehende Dinitrodibenzyl aufweisen, da sonst dessen Isolierung erschwert ist und die Oxidationsreaktion sehr leicht, insbesondere bei Verwendung von überschüssigen Basenmengen, bis zur Stufe des Dinitrostilbens weiterlaufen kann. Ebenso wirkt sich eine geringere Säurestärke des Lösungsmittels positiv auf den Reaktionsverlauf aus. Bevorzugt sind deshalb als organische Lösungs- und/oder Verdünnungsmittel Alkohole, wie Methanol, Ethanol, Propanol, iso-Propanol, n-Butanol, iso-Butanol, sec.-Butanol, tert.-Butanol tert.-Amylalkohol, Ethylenglykolmonomethylether, Ethylenglykolmonobutylether, Diethylenglykol und/oder Diethylenglykolmonomethylether, besonders bevorzugt tert.-Butanol, tert.-Amylalkohol, Methanol, Ethylenglykolmonomethylether, Ethylenglykolmonobutylether, Diethylenglykol und/oder Diethylenglykolmonomethylether, sowie Ether, wie Diethylether, Di-isopropylether, Dibutylether, tert.-Butylmethylether, Tetrahydrofuran, 1,4-Dioxan und/oder 1,3-Dioxan, besonders bevorzugt tert.-Butyl-methylether, Tetrahydrofuran oder 1,4-Dioxan.

Ebenfalls geeignet sind gegebenenfalls substituierte Carbonsäureamide, wie N,N-Dimethylformamid, N,N-Dimethylacetamid und/oder N-Methylpyrrolidon. Als weitere Lösungs- und/oder Verdünnungsmittel können noch Carbonsäureester und/oder Ketone, wie Essigsäureethylester, Essigsäurebutylester, Ethylenglykolmonomethyletheracetat, Aceton, Methylethylketon und/oder Cyclohexanon, eingesetzt werden. Kohlenwasserstoffe, wie Pentan, Hexan, Petrolether, Waschbenzin, Benzol, Toluol und/oder Xylol werden wegen der geringen Wasserlöslichkeit bevorzugt in Kombination mit wassermischbaren Lösungsmitteln, wie Methanol, eingesetzt.

Halogenierte Kohlenwasserstoffe können nur dann eingesetzt werden, wenn das enthaltende Halogen unter den Reaktionsbedingungen nicht durch Basen als Hydrohalogen abgespalten werden kann. Genannt seien z. B. Chlorbenzol, o-Dichlorbenzol, m-Dichlorbenzol und Chlortoluol.

Für die erfindungsgemäße Umsetzung werden bevorzugt die obengenannten Alkohole, besonders bevorzugt tert.-Butanol oder dessen Gemisch mit Methanol, eingesetzt.

Als Alkali- oder Erdalkalialkoholate werden in das erfindungsgemäße Verfahren üblicherweise solche eingesetzt, die sich von offenkettigen, verzweigten oder cyclischen niederen aliphatischen Alkoholen mit 1 bis 8 Kohlenstoffatomen, vorzugsweise 1 bis 5 Kohlenstoffatomen, ableiten. Diese Alkoholate werden bevorzugt als entsprechende alkoholische Lösungen eingesetzt. Bevorzugt werden die Natrium- oder Kaliumalkoholate des Alkohols in das erfindungsgemäße Verfahren eingesetzt, der auch als Reaktionsmedium Verwendung findet, wie Natriummethanolat oder Kalium-tert.-butanolat. Die Herstellung der Alkali- oder Erdalkalialkoholate ist bekannt und beispielsweise in Houben-Weyl, Methoden der organischen Chemie, 4. Auflage, Band VI/2, S. 1ff beschrieben.

Als Oxidationsmittel werden in das erfindungsgemäße Verfahren wäßrige Lösungen von unterhalogenigen Säuren und/oder deren Salzen, elementares Chlor oder Brom, wäßrige Lösungen von Wasserstoffperoxid und/oder dessen Salzen oder anorganische oder organische Persäuren und/oder deren Salzen eingesetzt. Als Lösungen von unterhalogenigen Säuren und/oder deren Salzen werden vorzugsweise die unter dem Trivialnamen Natronbleichlauge, Bleichlauge oder Chlorlauge bekannten Natriumhypochlorit-Lösungen eingesetzt, die durch Elektrolyse von Natriumchlorid-Lösungen oder durch Einleiten von Chlorgas in Natronlauge erhalten werden und einen Gehalt an aktivem Chlor von etwa 12,5 bis 13 Gew.-% aufweisen.

Als wäßrige Wasserstoffperoxid-Lösungen werden Lösungen mit einem Gehalt von etwa 5 bis 65 Gew.-% Wasserstoffperoxid, vorzugsweise 20 bis 50 Gew.-% Wasserstoffperoxid, besonders bevorzugt 30 bis 35 Gew.-% Wasserstoffperoxid, eingesetzt.

Als Persäuren bzw. deren Salze seien z. B. genannt: Perborate, wie Na-perborat, Perchlorate, wie K-perchlorat, Chlorate, Permanganate, Peroxomono- und -dicarbonate, Peroxomono- und -disulfate, Peroxophosphate sowie Perbenzoesäure.

Zur Herstellung der Dinitrodibenzyle werden die entsprechenden Nitrotoluole zunächst mit dem Alkoholat in einem organischen Lösungs- und/oder Verdünnungsmittel umgesetzt. Hierzu wird einer der Reaktionspartner, gegebenenfalls gelöst in einer Teilmenge oder der Gesamtmenge des organischen Lösungs- und/oder Verdünnungsmittels, vorgelegt und der andere Reaktionspartner, gelöst im Rest des organischen Lösungs- und/oder

Verdünnungsmittels, auf einmal oder portionsweise zugegeben. Die Reaktion verläuft exotherm, so daß gegebenenfalls gekühlt werden muß. Als günstig für die Durchführung der Reaktion hat sich ein Temperaturbereich von etwa 20 bis 70°C, vorzugsweise 30 bis 50°C, besonders bevorzugt von 35 bis 45°C, herausgestellt. Bei zu niedrigen Temperaturen verläuft die Reaktion zu langsam, bei zu hohen Temperaturen wird ein steigender Anteil an Dinitrostilben-Nebenprodukten gefunden.

Bei dem erfindungsgemäßen Verfahren wird im allgemeinen ein molarer Überschuß an Alkali- und/oder Erdalkalialkoholat eingesetzt. Üblicherweise werden etwa 1,05 bis 5 mol, bevorzugt 1,1 bis 3 mol pro mol 4-Nitrotoluol an Alkali- und/oder Erdalkalialkoholat eingesetzt. Die jeweils optimale Basen-Menge kann leicht durch einige Vorversuche ermittelt werden. Beispielsweise hat sich bei Einsatz von Natriummethanolat als Base eine etwa dreifache Molmenge, bewogen auf das 4-Nitrotoluol, als günstig erwiesen; bei Einsatz von Kalium-tert.-butanolat genügt ein geringerer (ca. bis 10 %) molarer Überschuß. Werden unterschüssige molare Mengen an Basen eingesetzt, so verringert sich die Ausbeute an 4,4'-Dinitrodibenzyl entsprechend (siehe Beispiel 3). Die Bildung des Carbanions (intensive Gelbfärbung) und der weiteren Radikal-Stufen (rot-blau-violette Färbung) verläuft im allgemeinen sehr schnell, so daß schon direkt nach der endgültigen Vermischung der Reaktionspartner oder nach einer nur kurzen Wartezeit die Oxidationsreaktion durchgeführt werden kann. Hierzu wird das Oxidationsmittel unter Rühren der basischen 4-Nitrotoluol- Lösung zugesetzt oder als Gas (Chlor) eingeleitet. Die Behandlung des Reaktionsgemisches mit Oxidationsmitteln wird vorzugsweise im gleichen Temperaturbereich wie die Umsetzung mit der Base durchgeführt. Die Reaktion ist ebenfalls exotherm und es muß gegebenenfalls gekühlt werden.

Die Menge an Oxidationsmittel beträgt beim erfindungsgemäßen Verfahren im allgemeinen 0,1 bis 2,0 mol, bevorzugt 0,5 bis 1,5 mol, bezogen auf 1 mol p-Nitrotoluol.

Die oxidative Dimerisierung verläuft im Falle der Chlorlauge spontan, d.h. direkt nach vollständiger Zugabe der Chlorlauge kann die Umsetzung beendet und aufgearbeitet werden. Im Falle von Wasserstoffperoxid, von Brom oder Chlor als Oxidationsmittel oder der anderen Oxidationsmittel sind kurze Nachreaktionszeiten von bis zu etwa 1/2 Stunde von Vorteil. Wegen der schnellen Umsetzung ist auch eine kontinuierliche Durchführung des Gesamtverfahrens möglich.

Nach Beendigung der Reaktion wird das Dibenzylprodukt nach üblichen Verfahren isoliert. Die Isolierung erfolgt vorzugsweise durch Wasserzugabe und Absaugen des ausgefallenen Produktes. Wegen der hohen Ausbeuten kann der Reaktionsansatz aber auch zunächst bis fast zur Trockne eingeengt werden und dann das Produkt mehrmals mit Wasser aufgeschlämmt und gewaschen werden. Die Trocknung erfolgt nach üblichen Standardverfahren, vorzugsweise in einem Vakuumtrockenschrank bei Temperaturen von etwa 50°C bei 20 bis 100 mbar. Die Aufarbeitung des Reaktionsansatzes wird erleichtet, wenn vor der Zugabe des Wassers durch Zugabe von Säuren, wie wäßriger Salzsäure oder Schwefelsäure, neutralisiert wird.

Das erfindungsgemäße Verfahren zeichnet sich gegenüber der bisher bekannten oxidativen Dimerisierung in Alkalihydroxid-Methanol-Gemischen dadurch aus, daß zum einen ein geringerer Basenüberschuß zur Herstellung der reaktiven Zwischenstufe erforderlich ist und zum anderen mit höheren Einsatzprodukt-Konzentrationen gearbeitet werden kann. Darüber hinaus verläuft die Oxidationsreaktion mit einer wäßrigen Hypochlorit-Lösung oder mit Chlor, Brom oder einer wäßrigen Lösung von Wasserstoffperoxid oder den anderen genannten Oxidationsmitteln sehr schnell, so daß insgesamt wesentlich höhere Raum-Zeit-Ausbeuten erzielt werden können als nach den Verfahren des Standes der Technik.

Daß bei dem erfindungsgemäßen Verfahren so hohe Ausbeuten an 4,4'-Dinitrodibenzylen erzielt werden können, überrascht besonders, da nach J. Chem. Soc. 91, S. 2079 (1907) eine Oxidation von 4-Nitrotoluol mit Hypochloritlösungen als nicht möglich angesehen wird.

Die nachfolgenden Beispiele sollen das erfindungsgemäße Verfahren verdeutlichen, ohne es jedoch auf diese Beispiele einzuschränken.

**Beispiel 1**

| | |
|---|---|
| 68,5 g (0,5 mol) | 4-Nitrotoluol werden in |
| 200 ml | tert.-Butanol bei 40°C vorgelegt. |
| 67,2 g (0,6 mol) | Kalium-tert.-butanolat, gelöst in |
| 400 ml | tert.-Butanol werden innerhalb von 5 min bei 40°C unter Rühren zugesetzt (sofortiger Farbumschlag nach intensiv gelb, orange, rot violett). Dann wird noch 10 min bei 40°C nachgerührt. Unter Rühren werden bei 40°C |
| a) 19,5 g (0,275 mol) | Chlorgas innerhalb von 1 h eingeleitet, oder |
| b) 48 g (0,30 mol) | Brom innerhalb von 20 min Zugetropft, oder |
| c) 26,7 g (0,275 mol) | 35 % wäßr. Wasserstoffperoxidlösung innerhalb von 15 min. zugetropft, oder |
| d) 150 g (0,55 mol Cl) | Chlorlauge mit 13 % aktivem Chlor, oder eine Lösung von |

| | |
|---|---|
| e) 67,6 g (0,25 mol) | Kaliumpersulfat ($K_2S_2O_8$) in |
| 600 ml | Wasser von 40°C innerhalb von 15 min zugetropft. Dann wird 30 min bei 4°C nachgerührt. |
| | Nach Zugabe von |
| 500 ml | Wasser wird der anfallende Feststoff abgesaugt, mit Wasser neutral gewaschen und bei 5°C getrocknet. |

| Nr. | a | b | c | d | e |
|---|---|---|---|---|---|
| Ausbeute | 65,5 g | 60,9 g | 63,87 g | 55,6 g | 56,6 g |
| Fp. | 174 - 225°C | 174 - 225°C | 174 - 225°C | 174 - 225°C | 172 - 225°C |

relative Zusammensetzung laut HPLC:

| | | | | | |
|---|---|---|---|---|---|
| 4-Nitrotoluol | 1,6 % | 1,6 % | 1,8 % | 2,9 % | 2,4 |
| Dinitrodibenzyl | 84,2 % | 91,3 % | 87,5 % | 90,0 % | 91,5 |
| Dinitrostilben | 10,5 % | 4,2 % | 7,7 % | 4,9 % | 0,6 |

Beispiel 1 zeigt die grundsätzliche Eignung der Oxidationsmittel Chlor, Brom, Chlorlauge, Wasserstoffperoxid/Wasser und K-persulfat für die oxidative Dimerisierung von 4-Nitrotoluol und demonstriert die hohen Umsetzungsgeschwindigkeiten mit den erfindungsgemäßen Basen und Oxidationsmitteln.

**Vergleichsversuch**

Zum Vergleich wurde ein Versuch durchgeführt, bei dem nach der Vorreaktion mit dem Kalium-tert.-butanolat 5 h lang Luft bei 40°C durch die Reaktionsmischung geleitet wurde. Die Aufarbeitung erfolgte wie in Beispiel 1 a - d beschrieben.

| | |
|---|---|
| Ausbeute: | 67,7 g |
| Fp.: | 230 - > 280°C |

| | |
|---|---|
| relative Zusammensetzung laut HPLC: | |
| 4-Nitrotoluol | - |
| Dinitrodibenzyl | 31,7 % |
| Dinitrostilben | 59,0 % |

Der Vergleichsversuch zeigt, daß zwar bei der Verwendung von Alkoholaten als Basen die Konzentration der Reaktionslösung an Ausgangsprodukt erhöht werden kann, daß aber für die Oxidation mit Luftsauerstoff erheblich längere Reaktionszeiten als mit den erfindungsgemäßen Oxidationsmitteln notwendig sind. Außerdem zeigt sich, daß bei gleicher Temperatur die Tendenz zur Bildung des Dinitrostilbenderivates beim Einsatz von Luftsauerstoff als Oxidationsmittel erheblich größer ist, d.h., daß die Reaktion bei niedrigeren Temperaturen (unter Kühlung) durchgeführt werden müßte.

**Beispiel 2**

| | |
|---|---|
| 411 g (3 mol) | 4-Nitrotoluol werden in |
| 1500 ml | tert.-Butanol bei 40°C vorgelegt. |
| 1620 g (9 Mol) | einer 30 %-igen Lösung von Natriummethanolat in Methanol werden innerhalb von 20 min unter Rühren zugegeben, wobei die Temperatur durch Kühlung auf 35 - 40°C gehalten wird. 15 min nach beendeter Zugabe werden |
| 154 g (1,59 mol) | einer 35 %-igen Wasserstoffperoxidlösung innerhalb von 30 min unter Rühren zugetropft, wobei die Temperatur durch leichte Kühlung auf 40°C gehalten wird. Es fällt nach und nach ein gelb-oranger Niederschlag aus. Nach beendeter Zugabe wird noch 1 h bei 40°C nachgerührt. Das Reaktionsgemisch wird auf ca. 20°C abgekühlt, mit halbkonzentrierter Salzsäure neutralisiert (pH = 7) und mit 1000 ml Wasser versetzt. Das ausgefallene Produkt wird abgesaugt, mit Wasser gewaschen und getrocknet. |

| | |
|---|---|
| Ausbeute: | 340,3 g |
| Fp.: | 165 - 228°C |
| relative Zusammensetzung laut HPLC: | |
| 4-Nitrotoluol: | 3,0 % |
| Dinitrodibenzyl: | 82,3 % |
| Dinitrostilben: | 5,3 %. |

In diesem Beispiel wird ein tert.-Butanol/Methanol-Gemisch als Reaktionsmedium eingesetzt. Das Beispiel belegt, daß auch größere Ansätze ohne Probleme (Kühlung) durchzuführen sind.

**Beispiel 3**

| | |
|---|---|
| 68,5 g (0,5 mol) | 4-Nitrotoluol werden bei 40°C vorgelegt. Unter Rühren wird eine Lösung von |
| 33,6 g (0,3 mol) | Kalium-tert.-butanolat in |
| 650 ml | tert.-Butanol bei 40°C innerhalb von 2 min zugesetzt. Dann wird 10 min bei 40°C weitergerührt. |
| a) 26,7 g (0,275 mol) | einer 35 %-igen Wasserstoffperoxidlösung bzw. |
| b) 150 g | Chlorlauge mit 13 % aktivem Chlor werden unter Rühren bei 40°C innerhalb von 15 min zugetropft. Dann wird 30 min bei 40°C nachgerührt, auf 20°C abgekühlt, mit Wasser versetzt, der Feststoff abgesaugt, mit Wasser neutral gewaschen und getrocknet. |

| | | |
|---|---|---|
| Ausbeute: | 37,29 g | 38,1 g |
| Fp.: | 165 - 225°C | 163 - 225°C |
| relative Zusammensetzung laut HPLC: | | |
| 4-Nitrotoluol: | 2,6 % | 2,9 % |
| Dinitrodibenzyl: | 91,5 % | 94,2 % |
| Dinitrostilben: | 1,4 % | 1,3 % |

Beispiel 3 zeigt, daß bei Verwendung der erfindungsgemäßen Oxidationsmittel die Ausbeute abhängig von der eingesetzten Basenmenge ist (Vergleich mit Beispiel 1c und 1d).

**Beispiel 4**

| | |
|---|---|
| 68,5 g (0,5 mol) | 4-Nitrotoluol werden bei 40°C vorgelegt. Unter Rühren wird eine Mischung aus |
| 67,2 g (0,6 mol) | Kalium-tert.-butanolat in |
| 400 ml | tert.-Butanol bei 40°C innerhalb weniger min zugegeben. Sofort hiernach werden |
| 150 g | Chlorlauge mit 13 % aktivem Chlor bei 40°C unter Rühren innerhalb von 15 min zugetropft. Es fällt sofort ein gelboranger Niederschlag aus. Dann wird noch 30 min bei 40°C gerührt, auf 20°C abgekühlt, mit |
| 500 ml | Wasser versetzt, das Produkt abgesaugt, mit Wasser neutral gewaschen und getrocknet. |

| | |
|---|---|
| Ausbeute: | 53,0 g |
| Fp.: | 160 - 215°C |
| relative Zusammensetzung laut HPLC: | |
| 4-Nitrotoluol: | 2,5 % |
| Dinitrodibenzyl | 88,4 % |
| Dinitrostilben: | 2,7 % |

Beispiel 4 zeigt, daß die Umsetzung der Base mit dem 4-Nitrotoluol sehr schnell abläuft, so daß die ebenfalls momentan ablaufende Oxidationsreaktion praktisch gleich anschließend durchgeführt werden kann, was für eine kontinuierliche Verfahrensweise sehr wichtig ist.

**Beispiel 5**

Das Beispiel 5 wird in der gleichen Weise wie Beispiel 1d durchgeführt mit folgender Abänderung:

Nach dem Zusatz der Chlorlauge und 30 minütigem Rühren bei 40°C wird der gesamte Ansatz an einem Rotationsverdampfer bei 40°C Badtemperatur bis fast zur Trockne eingeengt, wobei ein klares Gemisch aus Wasser und tert.-Butanol überdestilliert. Der Rückstand wird mit heißem Wasser aufgenommen, abgesaugt, 3 x mit heißem Wasser gewaschen und getrocknet.

| | |
|---|---|
| Ausbeute: | 65,6 g |
| Fp.: | 170 - 223°C |
| relative Zusammensetzung laut HPLC: | |
| 4-Nitrotoluol: | 7,6 % |
| Dinitrodibenzyl: | 78,4 % |
| Dinitrostilben: | 9,4 %. |

**Beispiel 6**

Das Beispiel 6 wird in der gleichen Weise wie Beispiel 1c durchgeführt mit folgender Abänderung:

Nach dem Zusatz der Wasserstoffperoxidlösung und 30 minütigem Rühren bei 40°C wird der gesamte Ansatz an einem Rotationsverdampfer bei 40°C Badtemperatur bis fast zur Trockne eingeengt, wobei ein klares Gemisch aus Wasser und tert.-Butanol überdestilliert. Der Rückstand wird mit heißem Wasser aufgenommen, abgesaugt, 3 x mit heißem Wasser gewaschen und getrocknet.

| | |
|---|---|
| Ausbeute: | 66,8 g |
| Fp.: | 172 - 223°C |
| relative Zusammensetzung laut HPLC: | |
| 4-Nitrotoluol: | 1,4 % |
| Dinitrodibenzyl: | 86,6 % |
| Dinitrostilben: | 9,3 %. |

Beispiel 5 und 6 zeigen, daß die Ausbeuten wesentlich erhöht werden können, wenn ein Teil des Reaktionsmediums vor der Aufarbeitung entfernt wird, d.h., daß Ausbeuteverluste durch die Löslichkeit der Produkte im Reaktionsmedium auftreten.

**Beispiel 7** Temperaturvariationen

| | |
|---|---|
| 68,5 g (0,5 mol) | 4-Nitrotoluol werden bei der Temperatur X°C vorgelegt, |
| 67,2 g (0,6 mol) | Kalium-tert.-butanolat in |
| 650 ml | tert.-Butanol werden in ca. 10 min unter Rühren zugegeben, wobei die Temperatur auf X°C gehalten wird. Nach beendeter Zugabe wird 10 min nachgerührt. Unter Rühren werden innerhalb ca. 15 min |
| 26,7 g (0,275 mol) | einer 35 %-igen Wasserstoffperoxidlösung zugesetzt, wobei die Temperatur auf Y°C gehalten wird. Es wird noch 30 min bei Y°C weitergerührt. Das Reaktionsgemisch wird auf ca. 20°C abgekühlt, mit 500 ml Wasser versetzt, der Niederschlag abgesaugt, mit Wasser neutral gewaschen und getrocknet. |

| | a | b | c |
|---|---|---|---|
| X | 40°C | 30°C | 55°C |
| Y | 55°C | 40°C | 40°C |

| | | | |
|---|---|---|---|
| Ausbeute | 62,5 g | 61,6 g | 65,0 g |
| Fp. | 188 - 245°C | 178 - 210°C | 165 - 228°C |
| relative Zusammensetzung laut HPLC: | | | |
| 4-Nitrotoluol | 0,8 % | 1,2 % | 2,1 % |
| Dinitrodibenzyl | 78,2 % | 90,8 % | 86,4 % |
| Dinitrostilben | 16,4 % | 5,5 % | 7,5 % |

In diesem Beispiel wird der Einfluß der Reaktionstemperatur der Basenreaktion und der Oxidationsreaktion auf die Produktzusammensetzung dargelegt. Eine niedrigere Temperatur bei der Basenumsetzung führt zu höheren Anteilen an Dinitrodibenzyl im Endprodukt, allerdings auf Kosten einer verminderten Ausbeute (Vergleich Beispiel 1c). Höhere Temperaturen bei der Oxidationsreaktion führen zu einer Erhöhung des Dinitrostilben-anteils im Endprodukt.

**Beispiel 8**

| | |
|---|---|
| 68,2 g (0,6 mol) | Kalium-tert.-butanolat werden in |
| 650 ml | tert.-Butanol gelöst, wobei die Temperatur auf 38°C steigt. Das Gemisch wird abgekühlt auf 23°C und unter Rühren auf einmal mit |
| 68,5 g (0,5 mol) | pulverisiertem 4-Nitrotoluol versetzt, wobei die Temperatur auf 30°C steigt. Ohne weitere Heizung oder Kühlung wird 10 min weitergerührt. |
| 150 g | Chlorlauge mit 13 Gew.-% aktivem Chlor werden innerhalb 15 min unter Rühren ohne Kühlung zugetropft, wobei die Temperatur auf 44°C steigt. Nach 30 min Rühren werden |
| 500 ml | Wasser zugesetzt, das ausgefallene Produkt abgesaugt, mit Wasser neutral gewaschen und getrocknet. |

| | |
|---|---|
| Ausbeute: | 44,4 g |
| Fp.: | 168 - 175°C |
| relative Zusammensetzung laut HPLC: | |
| 4-Nitrotoluol: | 2,5 % |
| Dinitrodibenzyl: | 93,0 % |
| Dinitrostilben: | - |

Beispiel 8 zeigt, daß es unter geeigneter Reaktionsführung möglich ist, den Dinitrostilbenanteil im Endprodukt auf einen chromatographisch nicht mehr nachweisbaren Betrag zu senken.

**Beispiel 9**

| | |
|---|---|
| 27,4 g (0,2 mol) | 4-Nitrotoluol werden bei 40°C vorgelegt, |
| 28,0 g (0,25 mol) | Kalium-tert.-butanolat werden in |
| 250 ml | eines Lösungsmittels gelöst bzw. dispergiert unter Rühren bei 40°C zugegeben. Es wird noch 10 min bei 40°C nachgerührt. Dann werden unter Rühren |
| 60 g | Chlorlauge mit 13 % aktivem Chlor innerhalb von 15 min zugesetzt. Nach weiteren 30 min Rühren bei 40°C werden |
| 200 ml | Wasser zugesetzt, der ausgefallene Feststoff abgesaugt, mit Wasser neutral gewaschen und bei 50°C getrocknet. |

| Lösungsmittel | a Tetrahydro-furan | b N,N-Dimethyl-formamid | c tert.-Butyl-methylether |
|---|---|---|---|
| Ausbeute | 12,0 g | 25,4 g | 19,8 g |
| Fp. | 165 - 228°C | 230 - 275°C | 170 - 267°C |
| relative Zusammensetzung laut HPLC: | | | |
| 4-Nitrotoluol | 3,8 % | 6,0 % | 3,4 % |
| Dinitrodibenzyl | 91,4 % | 31,3 % | 89,8 % |
| Dinitrostilben | 1,6 % | 57,5 % | 2,6 % |

Beispiel 9 zeigt den Lösungsmitteleinfluß auf die oxidative Dimerisierung des 4-Nitrotoluols. Lösungsmittel mit geringer Polarität und damit geringer Löslichkeit für das Alkoholat (Beispiel a und c) führen zu geringen Ausbeuten mit allerdings hohem Anteil an Dinitrodibenzyl.

Lösungsmittel mit hoher Löslichkeit für Base, Ausgangsprodukt und Endprodukt führen in hoher Ausbeute zu Produkten, die einen beträchtlichen Anteil an Dinitrostilben aufweisen (Beispiel b).

**Beispiel 10**

| | |
|---|---|
| | Zu einem Gemisch aus |
| 67,2 (0,6 mol) | Kalium-tert.-butanolat in |
| 150 ml | tert.-Butanol werden bei 40°C unter Rühren innerhalb von ca. 5 min |
| 75,5 g (0,5 mol) | 1,2-Dimethyl-4-nitrobenzol, gelöst in |
| 150 ml | tert.-Butanol zugetropft. Es wird noch 10 min weitergerührt und danach unter Rühren bei 40°C |
| 150 g | Chlorlauge mit 13 % aktivem Chlor innerhalb von 15 min zugesetzt. Nach 30 min werden |
| 500 ml | Wasser zugesetzt, das ausgefallene Produkt abgesaugt, mit Wasser neutral gewaschen und getrocknet. |

| | |
|---|---|
| Ausbeute: | 67,0 g |
| Fp.: | 198 - 212°C |
| relative Zusammensetzung laut HPLC: | |
| 1,2-Dimethyl-4-nitrobenzol: | 5,5 % |
| Dinitrodibenzyl: | 92,2 % |
| Dinitrostilben: | - |

Bei der etwas weniger aktivierten Verbindung 1,2-Dimethyl-4-nitrobenzol verläuft die oxidative Dimerisierung mindestens gleich gut wie beim 4-Nitrotoluol. Als praktisch einziges Produkt entsteht das entsprechende Dinitrodibenzyl.

**Beispiel 11**

| | |
|---|---|
| 85,75 g (0,5 mol) | 2-Chlor-4-nitrotoluol werden in |
| 100 ml | tert.-Butanol bei 40°C vorgelegt. Unter Rühren wird eine Mischung aus |
| 67,2 g (0,6 mol) | Kalium-tert.-butanolat mit |
| 300 ml | tert.-Butanol innerhalb von 5 min zugetropft, wobei die Temperatur auf 40°C gehalten wird. Nach beendeter Zugabe wird 10 min weitergerührt. Dann werden |
| 150 g | Chlorlauge mit 13 % aktivem Chlor unter Rühren innerhalb 15 min zugetropft und die Temperatur hierbei bei 40°C gehalten. Nach weiteren 30 min Nachrühren bei 40°C wird auf 20°C abgekühlt und |
| 500 ml | Wasser zugesetzt. Der Niederschlag wird abgesaugt, mit Wasser neutral gewaschen und im Trockenschrank getrocknet. |

| | |
|---|---|
| Ausbeute: | 85,5 g |
| Fp.: | 230 - 265°C |
| relative Zusammensetzung laut HPLC: | |
| 2-Chlor-4-nitrotoluol: | 11,3 % |
| Dinitrodibenzyl: | 55,0 % |
| Dinitrostilben: | 23,2 % |

Das 2-Chlor-4-nitrotoluol ist für die oxidative Dimerisierung stärker aktiviert als das 4-Nitrotoluol (-I-Effekt des Cl-Atoms). Deshalb tritt hier auch das entsprechende Dinitrostilben in größerer Menge als Nebenprodukt auf.

**Beispiel 12**

200 g eines nach Beispiel 1b erhaltenen Produkts, welches laut HPLC folgende relative Zusammensetzung besaß:

1,7 % 4-Nitrotoluol
92,4 % Dinitrodibenzyl
5,9 % Dinitrostilben,

wurden in 1800 ml Methanol aufgeschlämmt, mit 30 g Raney-Nickel versehen und bei 60 - 80°C unter einem $H_2$-Druck von 40 - 60 bar hydriert. Das erhaltene Gemisch wird heiß vom Raney-Nickel abfiltriert, bis auf etwa 1/3 bis 1/2 seines ursprünglichen Volumens eingeengt und mit 1 Liter Wasser versetzt. Es fällt ein perlmuttartig glänzender Niederschlag aus, der abgesaugt, mit Wasser gewaschen und getrocknet wird. Es handelt sich um 4,4'-Diaminodibenzyl, das laut HPLC eine Reinheit von ≥ 98 % aufweist.

Ausbeute: 132,7 g (85,1 % der Theorie bezogen auf Gesamtgewicht des Ausgangsproduktes)
Fp.: 126 - 130°C

**Patentansprüche**

1. Verfahren zur Herstellung von 4,4'-Dinitrodibenzylen der Formel

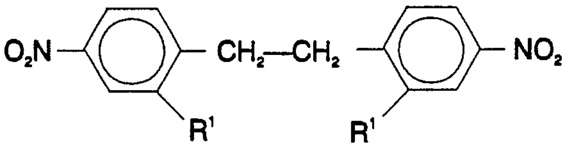 ,

worin

R1 Wasserstoff, Alkyl, Aryl, Alkaryl, Aralkyl oder Halogen bedeutet,

dadurch gekennzeichnet, daß man 4-Nitrotoluole der Formel

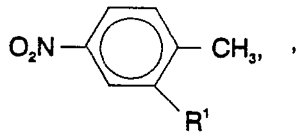

worin

$R^1$ die obengenannte Bedeutung besitzt,

in Gegenwart eines organischen Lösungs- und/oder Verdünnungsmittels mit einem Alkali- und/oder Erdalkalialkoholat umsetzt und anschließend das Reaktionsgemisch mit einer wäßrigen Lösung von unterhalogenigen Säuren und/oder deren Salzen oder mit Chlor, Brom oder einer wäßrigen Lösung von Wasserstoffperoxid und/oder dessen Salzen oder mit anorganischen oder organischen Persäuren und/oder deren Salzen behandelt.

2. Verfahren nach Anspruch 1, dadurch gekennzeichnet, daß man als organische Lösungs- und/oder Verdünnungsmittel Alkohole, Carbonsäureamide, Carbonsäureester, Ketone und/oder gegebenenfalls halogenierte Kohlenwasserstoffe einsetzt.

3. Verfahren nach Ansprüchen 1 und 2, dadurch gekennzeichnet, daß man als organisches Lösungs- und/oder Verdünnungsmittel tert.-Butanol, gegebenenfalls im Gemisch mit Methanol, einsetzt.

4. Verfahren nach Ansprüchen 1 bis 3, dadurch gekennzeichnet, daß man als Alkali- und/oder Erdalkalialkoholate solche einsetzt, die sich von offenkettigen, verzweigten oder cyclischen niederaliphatischen Alkoholen mit 1 bis 8 Kohlenstoffatomen ableiten.
5. Verfahren nach Ansprüchen 1 bis 4, dadurch gekennzeichnet, daß man als Alkali- und/oder Erdalkalialkoholate Natriummethanolat und/oder Kalium-tert.-butanolat einsetzt.

6. Verfahren nach Ansprüchen 1 bis 5, dadurch gekennzeichnet, daß man als unterhalogenige Säuren und/oder deren Salze Natriumhypochlorit-Lösungen einsetzt, die einen Gehalt an aktivem Chlor von 12,5 bis 13 Gew.-% aufweisen.

7. Verfahren nach Ansprüchen 1 bis 6, dadurch gekennzeichnet, daß man einen molaren Überschuß an Alkali- und/oder Erdalkalialkoholaten einsetzt.

8. Verfahren nach Ansprüchen 1 bis 7, dadurch gekennzeichnet, daß man 0,1 bis 2,0 mol Oxidationsmittel pro mol p-Nitrotoluol einsetzt.

## Claims

1. A process for the production of dinitrobenzyls corresponding to the following formula

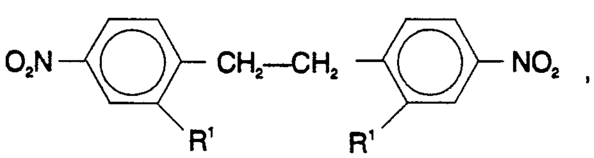

in which

$R^1$ is hydrogen, alkyl, aryl, alkaryl, aralkyl or halogen, characterized in that 4-nitrotoluenes corresponding to the following formula

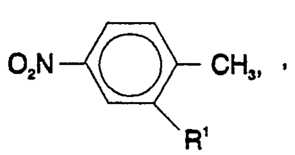

in which

$R^1$ is as defined above,

are reacted with an alkali and/or alkaline earth alcoholate in the presence of an organic solvent and/or diluent and the reaction mixture is subsequently reacted with an aqueous solution of hypohalous acids and/or salts thereof or with chlorine, bromine or an aqueous solution of hydrogen peroxide and/or salts thereof or with inorganic or organic per acids and/or salts thereof.

2. A process as claimed in claim 1, characterized in that alcohols, carboxylic acid amides, carboxylic acid esters, ketones and/or optionally halogenated hydrocarbons are used as the organic solvents and/or diluents.

3. A process as claimed in claims 1 and 2, characterized in that tert.-butanol, optionally in admixture with methanol, is used as the organic solvent and/or diluent.

4. A process as claimed in claims 1 to 3, characterized in that the alkali and/or alkaline earth alcoholates used are those derived from open-chain, branched or cyclic, lower aliphatic alcohols containing 1 to 8 carbon atoms.

5. A process as claimed in claims 1 to 4, characterized in that sodium methanolate and/or potassium tert.-butanolate is/are used as the alkali and/or alkaline-earth alcoholates.

6. A process as claimed in claims 1 to 5, characterized in that sodium hypochlorite solutions containing from 12.5 to 13 % by weight active chlorine are used as the hypohalous acids and/or salts thereof.

7. A process as claimed in claims 1 to 6, characterized in that a molar excess of alkali and/or alkaline earth alcoholates is used.

8. A process as claimed in claims 1 to 7, characterized in that from 0.1 to 2.0 mol oxidizing agent is used per p-nitrotoluene.

**Revendications**

1. Procédé de production de 4,4'-dinitrodibenzyles de formule

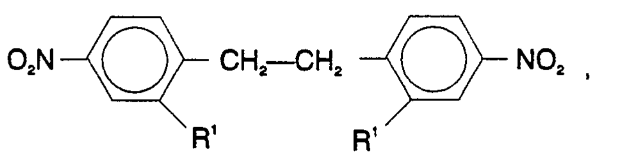

dans laquelle

$R^1$ désigne l'hydrogène, un groupe alkyle, aryle, alkaryle, aralkyle ou un halogène,
caractérisé en ce qu'on fait réagir des 4-nitrotoluènes de formule

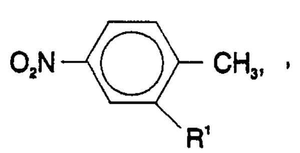

dans laquelle

$R^1$ a la définition indiquée ci-dessus,

en présence d'un solvant et/ou d'un diluant organique, avec un alcoolate alcalin et/ou alcalino-terreux puis on traite le mélange réactionnel avec une solution aqueuse d'acides hypohalogéneux et/ou de leurs sels ou avec du chlore, du brome ou une solution aqueuse de peroxyde d'hydrogène et/ou de ses sels ou avec des peracides inorganiques ou organiques et/ou leurs sels.

2. Procédé suivant la revendication 1, caractérisé en ce qu'on utilise comme solvants et/ou diluants organiques des alcools, des amides d'acides carboxyliques, des esters d'acides carboxyliques, des cétones et/ou des hydrocarbures éventuellement halogénés.

3. Procédé suivant les revendications 1 et 2, caractérisé en ce qu'on utilise comme solvants et/ou comme diluants organiques le tertiobutanol, le cas échéant en mélange avec du méthanol.

4. Procédé suivant les revendications 1 à 3, caractérisé en ce qu'on utilise comme alcoolates alcalins et/ou alcalino-terreux des alcoolates qui sont dérivés d'alcools aliphatiques inférieurs à chaîne ouverte, ramifiés ou cycliques ayant 1 à 8 atomes de carbone.

5. Procédé suivant les revendications 1 à 4, caractérisé en ce qu'on utilise comme alcoolates alcalins et/ou alcalino-terreux du méthanolate de sodium et/ou du tertio-butanolate de potassium.

6. Procédé suivant les revendications 1 à 5, caractérisé en ce qu'on utilise comme acides hypohalogéneux et/ou leurs sels des solutions d'hypochlorite de sodium qui ont une teneur en chlore actif de 12,5 à 13 % en poids.

7. Procédé suivant les revendications 1 à 6, caractérisé en ce qu'on utilise un excès molaire d'alcoolates alcalins et/ou alcalino-terreux.

8. Procédé suivant les revendications 1 à 7, caractérisé en ce qu'on utilise 0,1 à 2,0 moles d'agent oxydant par mole de p-nitrotoluène.